# FASCICULE DE BREVET EUROPEEN

(11) **EP 3 638 054 B1**
(45) Date de publication et mention de la délivrance du brevet: **21.08.2024**
(21) Numéro de dépôt: 18745997.9
(22) Date de dépôt: 15.06.2018
(51) Int. Cl.: A23L 5/46, A61K 35/748, A23L 33/195

(54) **PROCÉDÉ D'EXTRACTION DE COMPOSÉS HYDROSOLUBLES À PARTIR DE MICROALGUES ET/OU DE CYANOBACTÉRIES**
VERFAHREN ZUR EXTRAKTION VON WASSERLÖSLICHEN VERBINDUNGEN AUS MIKROALGEN UND / ODER CYANOBAKTERIEN
METHOD FOR EXTRACTING WATER-SOLUBLE COMPOUNDS FROM MICROALGAE AND/OR CYANOBACTERIA

(30) Priorité: 15.06.2017 FR 1770630
(43) Date de publication de la demande: 22.04.2020
(73) Titulaire: Algama, 91000 Evry (FR); Université d'Avignon et des Pays de Vaucluse, 84029 Avignon Cedex 1 (FR)
(72) Inventeur: CHEMAT, Farid, 84310 Morieres-les-Avignon (FR); ABERT VIAN, Maryline, 30390 Aramon (FR); VERNES, Léa, 84130 Le Pontet (FR); FELICE, Thomas, 92140 Clamart (FR); GONCALVES ALVES, Mathieu, 75009 Paris-9E-Arrondissement (FR)
(74) Mandataire: Touroude, Magali Linda
(86) Numéro de dépôt international: PCT/FR2018/000172
(87) Numéro de publication internationale: WO 2018/229364

(56) Documents cités:
- WO-A1-2014/154472
- CN-A- 104 844 707
- CN-A- 105 820 237
- PARIMI N. S. ET AL.: "Optimization of protein extraction from Spirulina platensis to generate a potential co-product and a biofuel feedstock with reduced nitrogen content", FRONTIERS IN ENERGY RESEARCH, vol. 3, 30, 23 June 2015 (2015-06-23), pages 1 - 9, XP002776375
- FURUKI TAKAO ET AL: "Rapid and selective extraction of phycocyanin from Spirulina platensis with ultrasonic cell disruption", JOURNAL OF APPLIED PHYCOLOGY, KLUWER, DORDRECHT, NL, vol. 15, no. 4, 1 July 2003 (2003-07-01), pages 319 - 324, XP009502020, ISSN: 0921-8971, DOI: 10.1023/A:1025118516888
- HADIYANTO H ET AL: "Response surface optimization of ultrasound assisted extraction (UAE) of phycocyanin from microalgae Spirulina platensis", EMIRATES JOURNAL OF FOOD AND AGRICULTURE, vol. 28, no. 4, April 2016 (2016-04-01), pages 227 - 234, XP002776376
- WANG M ET AL: "Microalgal Cell Disruption via Ultrasonic Nozzle Spraying", APPLIED BIOCHEMISTRY AND BIOTECHNOLOGY ; PART A: ENZYME ENGINEERING AND BIOTECHNOLOGY, HUMANA PRESS INC, NEW YORK, vol. 175, no. 2, 5 November 2014 (2014-11-05), pages 1111 - 1122, XP035428928, ISSN: 0273-2289, [retrieved on 20141105], DOI: 10.1007/S12010-014-1350-Z
- KOTOVICZ VALESCA ET AL: "Pulsed hydrostatic pressure and ultrasound assisted extraction of soluble matter from mate leaves (Ilex paraguariensis): Experiments and modeling", SEPARATION AND PURIFICATION TECHNOLOGY, vol. 132, 30 April 2014 (2014-04-30), pages 1 - 9, XP029038396, ISSN: 1383-5866, DOI: 10.1016/J.SEPPUR.2014.04.036

## Description

La présente invention se rapporte au domaine de la valorisation de la biomasse notamment de la biomasse algale, plus précisément la présente invention a pour objet un procédé d'extraction de composés hydrosolubles à partir de microalgues et/ou de cyanobactéries, ainsi que le produit obtenu par ce procédé et ses applications notamment dans le domaine alimentaire ou comme compléments alimentaires.

Afin de faire face à l'augmentation de la demande alimentaire mondiale, de nombreux industriels s'orientent vers des ressources alternatives telles que les microalgues. Ces microorganismes, d'une grande biodiversité, représentent une source de biomasse unique. Leur production se différencie de celle des plantes maraichères et céréalières, cultivées sur des terres arables, par leur rendement surfacique très élevé.

Cultivées depuis plus de 30 ans, les microalgues constituent un produit alimentaire à part entière. En nutrition humaine et animale, deux approches de valorisation existent : la première consiste à viser une consommation de la microalgue entière, la deuxième concerne l'extraction, la transformation et le conditionnement de molécules bioactives issues de celles-ci. En France, seules trois espèces de microalgues sont autorisées pour une consommation humaine sans transformation et détiennent des propriétés nutritionnelles exceptionnelles : la spiruline ou *Arthrospira platensis,* la microalgue verte *Chlorella* et la diatomée *Odontella aurita.* Ces micro-organismes s'annoncent comme des éléments majeurs dans l'enjeu de l'alimentation mondiale, grâce à leur teneur unique en protéines et au faible impact environnemental de leur production : faible consommation en eau et en énergie.

En particulier, la spiruline a ainsi attiré, ces dernières années, l'attention d'un grand nombre de chercheurs et d'industriels. Elle est déjà très répandue sur le marché sous la forme séchée non transformée, pour sa consommation en tant que complément alimentaire.

La spiruline contenant un grand nombre de composés d'intérêt à forte valeur ajoutée, de nombreux chercheurs et industriels se sont intéressés à l'extraction de composés d'intérêt à partir de cette cyanobactérie. La biomolécule usuellement extraite à partir de la spiruline est la phycocyanine (PC), protéine pigmentaire bleue à activité antioxydante, représentant 20 % de son poids sec (Vonshak, 1997). Cet intérêt grandissant pour la phycocyanine s'explique notamment par son pouvoir colorant. Actuellement, les colorants alimentaires bleus utilisés en France sont synthétiques : bleu patenté V et bleu brillant ; ils figurent souvent parmi les ingrédients des bonbons et boissons... Le bleu patenté V serait à l'origine d'une amplification de l'hyperactivité chez les enfants et pourrait constituer un potentiel allergène, c'est pourquoi il a récemment été interdit aux Etats Unis, Canada et Australie. De ce fait, une attention particulière a été portée sur l'usage de colorants naturels, et en particulier de la phycocyanine. De nombreuses études portent ainsi sur l'extraction de cette protéine à partir de la spiruline. A l'échelle du laboratoire des procédés tels que la macération dans différents solvants, la lyse enzymatique, les ultrasons, sont employés. Ainsi les études bibliographiques détaillent plusieurs protocoles d'extraction de la PC à partir de la spiruline : emploi des ultrasons (20 kHz, 200W) dans du tampon phosphate 0.1 M et pH 6.8 (Furuki *et al,* 2003), macération dans du tampon phosphate durant 4h à température ambiante sous agitation continue (Chaiklahan et al, 2011), emploi de tampon Tris-HCl avec des lysozymes (Wenjun *et al,* 2013), macération à froid dans de l'eau distillée durant 24h (Kamble *et al,* 2013), macération dans du tampon sodium acétate à 30 °C durant 24h (Silveira *et al.,* 2007).

Afin de produire des extraits de PC en grande quantité, des procédés d'extraction à partir de spiruline ont été mis au point ou adaptés à l'échelle industrielle. On trouve ainsi des brevets protégeant les procédés d'extraction. Par exemple, le brevet WO 2014045177 A1 déposé en 2013 par Ecosystem protège un procédé d'extraction consistant à faire macérer la biomasse de spiruline durant 15 jours dans du glycérol puis d'employer une filtration frontale afin de récupérer le filtrat contenant la PC concentrée. Toutefois, ce procédé d'extraction par macération présente deux inconvénients : la durée d'extraction et le faible rendement. D'autres méthodes, dites d'« intensification », ont émergé ces dernières années afin de pallier ce problème : l'usage de microondes, ultrasons, fluides supercritiques (CO₂), lyse enzymatique... Les fluides supercritiques sont principalement employés pour extraire les composés hydrophobes, liposolubles tels que les caroténoïdes, tocophérols, acides gras... (Supercritical Carbon Dioxide and Microwave-Assisted Extraction of Functional Lipophilic Compounds from *Arthrospira platensis,* Esquivel-Hernández et al, 2016). Par ailleurs, plusieurs auteurs rapportent l'emploi d'ultrasons pour l'extraction de microalgue, et notamment de spiruline (Ultrasound assisted extraction of beta-carotene from Spirulina platensis, Dey and Rathod, 2013 _ Phyocyanin extraction from microalgae Spirulina platensis assisted by ultrasound irradiation : effect of time and température, Hadiyanto *et al.,* 2016). L'emploi d'ultrasons pour l'extraction de microalgue est également décrit dans CN104844707A, CN105820237A et Parimi et al. (Frontiers in Energy Research, Juin 2015, Volume 3, Article 30). Ces techniques dites d'« intensification » présentent l'avantage d'être plus rapides et efficaces en comparaison aux méthodes conventionnelles telles que la macération. Cependant, les techniques d'extraction par ultrasons classiques ne permettent pas l'obtention d'un extrait dont la composition en composés hydrosolubles soit modulable selon la nature et la quantité souhaitée pour chaque composé hydrosoluble.

Les procédés cités précédemment visent l'obtention d'extraits de spiruline « titrés » en phycocyanine pour des applications de colorant alimentaire naturel ou bien de complément alimentaire sous forme d'ampoules unitaires. Seuls quelques rares extraits de spiruline sont, à ce jour, destinés à être incorporés à des formulations alimentaires et servent uniquement à les colorer.

Chez certaines autres microalgues telles que Chlorella, qui est constituée de cellulose et possède une forte rigidité entraînant une résistance élevée de l'algue vis-à-vis des contraintes mécaniques, ces procédés ne permettraient pas d'extraire efficacement les composés hydrosolubles telles que la chlorophylle.

Les Demandeurs ont aujourd'hui mis au point un procédé qui permet de moduler les bulles de cavitation générées par les ultrasons, à l'origine de l'efficacité de l'extraction, par d'autres paramètres appliqués pendant le traitement des micro-algues aux ultrasons, tels que la pression, la température, et le débit, permettant ainsi une intensification et une modulation du procédé d'extraction de composés hydrosolubles, selon la nature et la quantité des composés hydrosolubles souhaités dans l'extrait.

Contrairement à ce qui existe déjà sur le marché, le procédé selon l'invention permet de générer de manière optimisée, à l'échelle industrielle, à partir d'une biomasse de microalgues de départ, d'une solution aqueuse et d'un procédé, différents extraits à haute valeur nutritionnelle, riches en composés hydrosolubles, en particulier protéines, vitamines hydrosolubles, chlorophylle, phycocyanine et minéraux, destiné à l'alimentation humaine lesdits extraits ayant des compositions modulables en lesdits composés hydrosolubles. Selon les paramètres de puissance ultrasonore (Pus), Température, Pression et débit choisis au départ, il est ainsi possible de faire varier la composition nutritionnelle de l'extrait mais également sa couleur.

Ainsi un premier objet de la présente invention concerne un procédé d'obtention de composés hydrosolubles à partir de microalgues eucaryotes (microalgues) ou procaryotes (cyanobactéries), caractérisé en ce que ledit procédé comprend au moins une étape d'extraction desdits composés hydrosolubles par un traitement d'ultrasons avec application d'une puissance ultrasonore (Pus) comprise entre 10 et 1000 W/L appliquée aux microalgues ou aux cyanobactéries en mélange avec une solutions aqueuse à laquelle est appliquée, de préférence de manière concomitante une pression (P) comprise entre 1 et 2 bars, une température (T) comprise entre 5 et 70°C, et un débit (D) compris entre 1 et 1000 L/heure.

Par microalgues, on entend désigner selon la présente invention les microalgues eucaryotes, qui sont caractérisée par une paroi cellulaire et un noyau, comprenant les chlorophytes, les chrysophytes et les pyrrophytes, lesdits microalgues eucaryotes étant communément dénommées « microalgues », et les microalgues procaryotes, qui ne possèdent pas de noyau et de paroi cellulaire, comprenant les cyanophytes, ci-après dénommées spécifiquement « cyanobactéries ».

De manière préférée selon l'invention, les microalgues eucaryotes sont choisies parmi les chlorophytes, de préférence parmi Chlorella, Nannocloropsis, Dunaliella et Euglena.

De manière préférée selon l'invention, les cyanobactéries sont choisies parmi la spiruline (Arthrospira platensis ou Spirulina maxima) et l'AFA (Aphanizomenon Floes-aquae).

De manière particulièrement préférée selon l'invention la microalgue selon l'invention est la spiruline
Par composés hydrosolubles, on entend désigner selon la présente invention des composés solubles dans l'eau et contenus dans les microalgues et/ou les cyanobactéries, de préférence choisi parmi les protéines et peptides, les vitamines hydrosolubles, de préférence les vitamines du groupe B (en particulier Thiamine (B1), Riboflavine (B2), Niacine (B3), Acide pantothénique (B5), pyridoxine (B6), Biotine (B8), Folate (B9), Cobalamine (B12)), les minéraux, de préférence le sodium, le calcium, le potassium, le magnésium et le fer, la C-phycocyanine (ou phycocyanine), et la chlorophylle.

De manière particulièrement préférée selon l'invention la microalgue selon l'invention est la spiruline et le composé hydrosoluble est la C-phycocyanine.

Par traitement d'ultrasons avec application d'une puissance ultrasonore (Pus) on entend désigner selon la présente invention un traitement physique mettant en oeuvre un réacteur à ultrasons. De manière préférée selon l'invention, la puissance ultrasonore est comprise entre 10 et 1000 W/L de solution aqueuse mélangée aux microalgues ou aux cyanobactéries, de manière préférée entre 30 et 100 W/L , de manière encore préférée entre 50 et 80 W/L. De manière préférée, l'énergie déployée par le réacteur à ultrasons varie entre 500 et 5000 J, le débit d'alimentation du réacteur à ultrasons varie de 1 L/heure à 1000L/heure, de préférence 1L/heure, et le traitement aux ultrasons est réalisé pendant une durée variant de 30 secondes à 90 minutes.

Par solution aqueuse on entend désigner selon la présente invention une solution aqueuse compatible pour un usage alimentaire. De manière préférée, on entend désigner une eau tamponnée, de préférence du tampon phosphate à pH 7, le tampon utilisé étant le sodium phosphate. En particulier, il est préférable que la solution aqueuse selon l'invention ne comprennent pas de conservateurs chimiques, ou encore de solvants organiques d'origine pétrochimique (méthanol, chloroform, hexane, ethanol) qui présentent une toxicité qui les rendraient incompatibles pour un usage alimentaire des composés hydrosolubles obtenus par le procédé selon l'invention. Il n'est pas souhaitable non plus d'utiliser un solvant acide car certains composés hydrosolubles comme la phycocyanine sont très sensibles au pH et toute variation important du pH, en dessous de pH5 et au-dessus de pH 7,5 entraîne sa précipitation. Un solvant alcoolique n'est pas préféré car certains composés hydrosolubles comme la phycocyanine perdent alors une grande partie de leurs propriétés anti-oxydantes.

De manière préférée selon la présente invention, le rapport microalgues ou cyanobactéries /solution aqueuse est compris entre 1/5^{ème} et 1/50^{ème}.

De manière concomitante à ce traitement ultrasonore et à ce débit est appliqué une pression (P) comprise entre 1 et 2 bars, et une température (T) comprise entre 5 et 70°C, de manière préférée entre 20 et 30°C. De manière particulièrement préférée, la température est maintenue constante entre 20 et 30°C pendant toute la durée du procédé d'extraction des composés hydrosolubles .

De manière préférée selon l'invention, le mélange microalgue/solution aqueuse est accueilli dans un réservoir à double paroi. Ledit réservoir peut être thermostaté grâce à une circulation d'eau et un thermostat permettant de régler la température souhaitée. Le mélange est ensuite envoyé grâce à une pompe et une vanne vers un réacteur à ultrasons comprenant une sonde ultrasonore reliée à un générateur d'ultrasons. Ledit réacteur à ultrasons peut lui aussi être préférentiellement doté d'une double paroi permettant d'être thermostaté grâce à une circulation d'eau et un thermostat permettant de régler la température souhaitée. La vanne permet d'imposer une résistance à la circulation du mélange et donc d'augmenter la pression subit par le mélange. De préférence, un manomètre permet de suivre, en amont du réacteur ultrasonore la pression exercée sur le mélange, et de moduler l'ouverture de la vanne pour appliquer la pression souhaitée sur le mélange.

Ainsi, de manière préférée selon l'invention, la température (T) est contrôlée grâce à un thermostat, de préférence via une circulation d'eau thermostatée dans la double paroi d'un réacteur comprenant le mélange microalgue/solution aqueuse, et le mélange est maintenu à la température souhaitée. Il est à noter que lors de l'étape de traitement aux ultrasons, avec éventuellement application d'une pression supérieure à 1 bar, la température du mélange va avoir tendance à augmenter, et que le thermostat peut avoir alors le rôle de maintenir une température constante du mélange. Le contrôle de la température permet d'éviter une élévation trop importante qui risquerait d'endommager les composés hydrosolubles sensibles à la chaleur, dits thermolabiles. La Demanderesse a mis en évidence que lorsque la température excède les 40°C certaines molécules d'intérêt comme la phycoyanine peuvent être masquées (par la chlorophylle par exemple). Par ailleurs la température optimale à l'extraction composés hydrosolubles d'intérêts se situe autour de 25°C. Ainsi avec une thermorégulation à 20°C, couplée à l'effet chauffant des ultrasons, une température avoisinant les 25-30 °C (proche de la température optimale d'extraction) est obtenue. Cependant, la modulation de la température permet d'extraire différents types de composés : à faible température l'extraction de composés thermolabiles est favorisée alors qu'à température élevée les composés sensibles à la chaleur sont masqués mais d'autres molécules plus difficiles à extraire à froid se retrouvent en solution comme la chlorophylle (de nature apolaire, soluble dans les solvants organiques en temps normal).

De manière préférée selon l'invention, la pression (P) est appliquée à l'aide d'une vanne sur le système permettant d'alimenter le réacteur où sera appliquée Pᵤₛ et permet d'imposer une résistance à la circulation du mélange microalgue/solution aqueuse dans le système. Plus la vanne est proche de la position fermée, plus l'écoulement est rendu difficile du fait du rétrécissement de l'orifice, ce qui provoque un ralentissement de la circulation du mélange microalgue/solution aqueuse dans le système ; or la pompe impose un débit constant ce qui provoque une élévation de la pression.

Selon un aspect préféré de la présente invention, la puissance ultrasonore, la pression, la température et le débit sont modulés pour faire varier les composés hydrosolubles extraits desdites microalgues eucaryotes ou cyanobactéries.

Ainsi, en modulant Pus, T, P et/ou D, différents extraits peuvent être produit à partir d'une même biomasse de microalgue ou de cyanobactérie de départ, avec une solution aqueuse donnée.

Ainsi, de manière préférée selon l'invention, plus la puissance ultrasonore, la pression et la température délivrées au milieu sont importantes, plus l'extrait sera riche en protéines et en chlorophylle. Au contraire, moins la puissance ultrasonore et la température sont importantes, moins l'extrait sera riche en composés hydrosolubles.

Ainsi par exemple on peut appliquer une puissance ultrasonore de 100 W/L avec une pression de 2 bars à une température contrôlée de 20°C pendant 10 min sur un mélange de spiruline et tampon phosphate 7 pour obtenir un extrait plus riche en chlorophylle , et donc de couleur verte plus intense que par un procédé avec une puissance ultrasonore de 30 W/L avec une pression de 1 bar à une température contrôlée de 20°C pendant 10 min qui sera plus bleu.

De manière préférée selon la présente invention, la durée de l'étape de traitement ultrasonore est comprise entre 1 à 90 minutes. La modulation de la durée permet de faire varier la composition en composés hydrosolubles, et donc également la concentration. Ceci s'explique par le fait que plus le mélange microalgue/solution aqueuse passe fréquemment dans le réacteur à ultrasons, plus il va être soumise aux ultrasons et libérer de composés hydrosolubles.

Selon un aspect préféré de la présente invention, la puissance ultrasonore, la pression, la température et le débit sont modulés pour faire varier la couleur de l'extrait obtenu par ledit procédé.

Ainsi, de manière préférée selon l'invention, plus la puissance ultrasonore, la pression et la température délivrées au milieu sont importantes, plus l'extrait sera riche en protéines et en chlorophylle et donc de couleur verte intense. Au contraire, moins la puissance ultrasonore et la température sont importantes, moins l'extrait sera riche en composés hydrosoluble et plus la couleur de l'extrait généré sera bleue (du fait de l'absence de chlorophylle).

Selon un aspect préféré de la présente invention, les étapes sont appliquées au mélange de microalgues eucaryotes ou cyanobactéries et de solution aqueuse en continu, de préférence à l'aide d'une pompe assurant la circulation du mélange dans un circuit fermé ou ouvert.

Selon un aspect préféré de la présente invention, le procédé d'obtention de composés hydrosolubles selon l'invention comprend en outre, une étape supplémentaire de séparation solide-liquide, de préférence par centrifugation, de l'extrait obtenu lors de l'étape d'extraction. Selon un aspect préféré de la présente invention, le procédé d'obtention de composés hydrosolubles selon l'invention comprend en outre une étape supplémentaire de filtration de la partie liquide obtenue par séparation de la partie solide (débris cellulaires) de l'extrait.

De manière préférée selon la présente invention, la filtration est effectuée de manière frontale, avantageusement sur filtre alimentaire, par exemple en polyamide, notamment en nylon, avec une finesse de 2 à 50 microns, de préférence inférieure à 25 microns.

Les produits tels qu'obtenus par le procédé selon l'invention renferment des composés hydrosolubles présents dans les microalgues ou cyanobactéries.

Plus particulièrement, ledit procédé permet d'extraire des microalgues et cyanobactéries les composés hydrosolubles, séparés de la fraction liposoluble de la microalgue ou de la cyanobactérie. Préférentiellement, l'extrait produit possédera des qualités organoleptiques ainsi qu'une composition nutritionnelle optimale.

Les produits tels qu'obtenus par le procédé selon l'invention peuvent être utilisés dans des compositions chimiques, alimentaires, cosmétiques ou pharmaceutiques.

Compte tenu des avantages évoqués ci-dessus présentés par ces filtrats/extraits, leurs applications dans le domaine alimentaire, médical ou cosmétique, y compris comme compléments alimentaires, s'avèrent particulièrement intéressantes. Les rétentats obtenus à l'issue de l'étape de filtration sont également utilisables dans le domaine alimentaire, médical ou cosmétique ou comme compléments alimentaires.

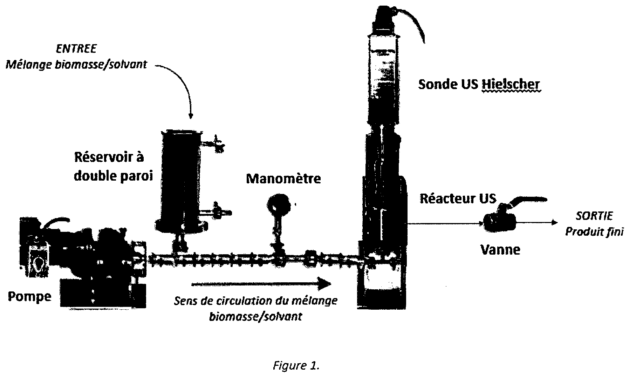

On décrira maintenant, à titre d'exemple non limitatif, un mode de réalisation de l'invention en référence aux dessins schématiques annexés dans lesquels la figure 1 est un schéma du dispositif de montage de mano-thermo-sonication (MTS) pour la mise en oeuvre du procédé selon l'invention, comprenant une sonde et un réacteur ultrasonore pour générer Pus, un thermostat pour régler T, une vanne et un manomètre pour régler P et une pompe pour régler D. On décrira maintenant, à titre d'exemple non limitatif, le séquencement du procédé selon l'invention

Ledit procédé se présente sous la forme d'un circuit fermé ou ouvert (selon le nombre de cycles choisi) permettant le traitement d'une solution de microalgues (5 % en MS) en continu. Description des différents éléments du montage MTS :
1) Le réservoir permet d'accueillir le mélange microalgue et solution aqueuse à traiter. Il s'agit d'un réservoir à double paroi pouvant être thermostaté grâce à une circulation d'eau.
2) Une pompe régule la circulation du mélange dans le système à débit constant.
3) La sonde ultrasonore est reliée à un générateur permettant de fixer la puissance ultrasonore souhaitée. La sonde est immergée dans le liquide à traiter au 2/3 et émet une fréquence fixe qui lui est propre (environ 20 kHz).
4) Le réacteur à ultrasons est doté d'une double paroi permettant de thermostater le mélange via une circulation d'eau thermostatée. La sonde ultrasonore pénètre le réacteur de manière à délivrer des ondes ultrasonores dans le milieu.
5) La vanne permet d'imposer une résistance à la circulation du mélange et donc d'augmenter la pression dans le système. Il est possible de suivre visuellement cette variation de pression sur le manomètre présent en amont du réacteur ultrasonore.

Une fois le mélange traité aux ultrasons dans le dispositif continu, une vidange du système est effectuée afin d'amener le mélange dans un module de centrifugation permettant une séparation solide/liquide entre les résidus de biomasse solides et la fraction hydrosoluble. Une étape de filtration permet d'obtenir un extrait clair et limpide.

### Exemples :

### Exemple 1 : US faible puissance, sans pression → extrait bleu

La biomasse (*Arthrospira platensis*) est mélangée à une solution aqueuse d'extraction (tampon phosphate pH 7) selon le ratio 1/20 ; soit 1 g de biomasse pour 20 g de solution aqueuse. Dans ce cas, nous introduisons 4 g de spiruline dans 80 mL de solution aqueuse. Le mélange est soumis aux ultrasons à faible puissance ultrasonore (30 W) durant 10 min, avec une thermorégulation à 20 °C. Aucune pression n'est imposée au système. Les extraits sont ensuite centrifugés 10 min à 8000 rpm puis filtré par filtration frontale sur Büchner (porosité 8 µm) afin de retirer des éventuelles particules en suspension.

L'extrait obtenu est de couleur bleue du fait de la présence de phycocyanine (8.34 g/100 g Matière Sèche (MS)) et contient 33.36 g/100 g MS de protéines.

### Exemple 2 : US forte puissance + pression → extrait vert riche en protéines

La biomasse (*Arthrospira platensis*) est mélangée à la solution aqueuse d'extraction (tampon phosphate pH 7) au ratio 1/20 ; soit 1 g de biomasse pour 20 g de solution aqueuse. Dans ce cas, nous introduisons 4 g de spiruline dans 80 mL de solution aqueuse. Le mélange est soumis aux ultrasons à puissance ultrasonore (100 W/L, soit environ 80 % d'amplitude) durant 10 min, avec une thermorégulation à 20 °C et une pression avoisinant les 2 bars. Les extraits sont ensuite centrifugés 10 min à 8000 rpm puis filtré par filtration frontale sur Büchner (porosité 8 µm) afin de retirer des éventuelles particules en suspension.

L'extrait obtenu est de couleur verte intense du fait de la présence de chlorophylle qui a été extraire du fait de l'intensité de la méthode. La phycocyanine est toujours présente dans l'extrait (11.47 g/100 g MS) et la teneur en protéines est de 46.19 %.

## Revendications

1. Procédé d'obtention de composés hydrosolubles à partir de microalgues eucaryotes ou procaryotes (cyanobactéries), **caractérisé en ce que** ledit procédé comprend au moins une étape d'extraction desdits composés hydrosolubles par un traitement d'ultrasons avec application d'une puissance ultrasonore (Pus) comprise entre 10 et 1000 W/L appliquée aux microalgues ou aux cyanobactéries en mélange avec un solution aqueuse à laquelle est appliquée, de manière concomitante, une pression (P) comprise entre 1 et 2 bars, une température (T) comprise entre 5 et 70°C, et un débit (D) compris entre 1 et 1000 L/heure.

2. Procédé selon la revendication 1 **caractérisé en ce que** la puissance ultrasonore, la pression, la température et le débit sont modulés pour faire varier les composés hydrosolubles extraits desdites microalgues eucaryotes ou cyanobactéries.

3. Procédé selon la revendication 1 ou 2 **caractérisé en ce que** la durée de l'étape est comprise entre 30 secondes et 90 minutes.

4. Procédé selon l'une quelconque des revendications précédentes **caractérisé en ce que** la puissance ultrasonore, la pression, la température et le débit sont modulés pour faire varier la couleur de l'extrait obtenu par ledit procédé.

5. Procédé selon l'une quelconque des revendications précédentes **caractérisé en ce que** les étapes sont appliquées au mélange de microalgues eucaryotes ou cyanobactéries et de solution aqueuse en continu, de préférence à l'aide d'une pompe assurant la circulation du mélange dans un circuit fermé ou ouvert.

6. Procédé selon l'une quelconque des revendications précédentes **caractérisé en ce que** les composés hydrosolubles extraits sont choisi parmi les protéines et peptides, les vitamines hydrosolubles, de préférence les vitamines du groupe B, les minéraux, de préférence le sodium, le calcium, le potassium, le magnésium et le fer, la C-phycocyanine, et la chlorophylle.

7. Procédé selon l'une quelconque des revendications précédentes **caractérisé en ce que** les microalgues eucaryotes sont choisies parmi Chlorella, Nannocloropsis, Dunaliella et Euglena, et que les cyanobactéries sont choisies parmi la spiruline (Arthrospira platensis ou Spirulina maxima) et l'AFA (Aphanizomenon Floes-aquae).

8. Procédé selon l'une quelconque des revendications précédentes **caractérisé en ce qu'**il comprend une étape supplémentaire de séparation solide-liquide, de préférence par centrifugation, de l'extrait obtenu.

9. Procédé selon la revendication 8 **caractérisé en ce qu'**il comprend une étape supplémentaire de filtration de la partie liquide obtenue par séparation de l'extrait.

10. Procédé selon l'une quelconque des revendications précédentes **caractérisé en ce que** le rapport microalgues ou cyanobactéries /solution aqueuse est compris entre 1/5^{ème} et 1/50^{ème}.

## Patentansprüche

1. Verfahren zum Gewinnen wasserlöslicher Verbindungen aus eukaryotischen oder prokaryotischen Mikroalgen (Cyanobakterien),
**dadurch gekennzeichnet, dass** das Verfahren mindestens einen Schritt eines Extrahierens der wasserlöslichen Verbindungen durch eine Ultraschallbehandlung unter Anwendung einer Ultraschallleistung (Pus) zwischen 10 und 1000 W/L umfasst, die auf Mikroalgen oder Cyanobakterien gemischt mit einer wässrigen Lösung angewendet wird, auf die gleichzeitig ein Druck (P) zwischen 1 und 2 bar, eine Temperatur (T) zwischen 5 und 70 °C und eine Durchflussrate (D) zwischen 1 und 1000 UStunde angewendet wird.

2. Verfahren nach Anspruch 1, **dadurch gekennzeichnet, dass** die Ultraschallleistung, der Druck, die Temperatur und die Durchflussrate zum Variieren der wasserlöslichen Verbindungen, die aus den eukaryotischen Mikroalgen oder Cyanobakterien extrahiert werden, moduliert werden.

3. Verfahren nach Anspruch 1 oder 2, **dadurch gekennzeichnet, dass** die Dauer des Schrittes zwischen 30 Sekunden und 90 Minuten beträgt.

4. Verfahren nach einem der vorstehenden Ansprüche,
**dadurch gekennzeichnet, dass** die Ultraschallleistung, der Druck, die Temperatur und die Durchflussrate zum Variieren der Farbe des Extrakts, der durch das Verfahren gewonnen wird, moduliert werden.

5. Verfahren nach einem der vorstehenden Ansprüche,
**dadurch gekennzeichnet, dass** die Schritte kontinuierlich auf die Mischung aus eukaryotischen Mikroalgen oder Cyanobakterien und wässriger Lösung angewendet werden, vorzugsweise mithilfe einer Pumpe, die die Zirkulation der Mischung in einem geschlossenen oder offenen Kreislauf gewährleistet.

6. Verfahren nach einem der vorstehenden Ansprüche,
**dadurch gekennzeichnet, dass** die extrahierten wasserlöslichen Verbindungen aus Proteinen und Peptiden, wasserlöslichen Vitaminen, vorzugsweise Vitaminen der Gruppe B, Mineralien, vorzugsweise Natrium, Kalzium, Kalium, Magnesium und Eisen, C-Phycocyanin und Chlorophyll ausgewählt werden.

7. Verfahren nach einem der vorstehenden Ansprüche,
**dadurch gekennzeichnet, dass** die eukaryotischen Mikroalgen aus Chlorella, Nannocloropsis, Dunaliella und Euglena ausgewählt werden und dass die Cyanobakterien aus Spirulina (Arthrospira platensis oder Spirulina maxima) und AFA (Aphanizomenon floes-aquae) ausgewählt werden.

8. Verfahren nach einem der vorstehenden Ansprüche,
**dadurch gekennzeichnet, dass** es einen zusätzlichen Schritt einer Fest-Flüssig-Trennung, vorzugsweise durch Zentrifugieren, des gewonnen Extrakts umfasst.

9. Verfahren nach Anspruch 8, **dadurch gekennzeichnet, dass** es einen zusätzlichen Schritt einer Filtration des flüssigen Teils umfasst, der durch Trennung des Extrakts gewonnen wird.

10. Verfahren nach einem der vorstehenden Ansprüche,
**dadurch gekennzeichnet, dass** das Verhältnis Mikroalgen oder Cyanobakterien/wässrige Lösung zwischen 1/5 und 1/50 liegt.

## Claims

1. A method for obtaining water-soluble compounds from eukaryotic or prokaryotic microalgae (cyanobacteria), **characterized in that** said method comprises at least one step of extracting said water-soluble compounds using an ultrasound treatment by applying an ultrasonic power (Pus) of between 10 and 1000 W/L to the microalgae or cyanobacteria mixed with an aqueous solution to which a pressure (P) of between 1 and 2 bar, a temperature (T) of between 5 and 70°C, and a flow rate (D) of between 1 and 1000 L/hour is concurrently applied.

2. The method according to claim 1, **characterized in that** the ultrasonic power, pressure, temperature and flow rate are modulated so as to vary the water-soluble compounds extracted from said eukaryotic microalgae or cyanobacteria.

3. The method according to claim 1 or claim 2, **characterized in that** the duration of the step is between 30 seconds and 90 minutes.

4. The method according to any of the preceding claims,
**characterized in that** the ultrasonic power, pressure, temperature and flow rate are modulated so as to vary the color of the extract obtained using said method.

5. The method according to any of the preceding claims,
**characterized in that** the steps are applied to the mixture of eukaryotic microalgae or cyanobacteria and aqueous solution continuously, preferably by means of a pump which ensures the circulation of the mixture in a closed or an open circuit.

6. The method according to any of the preceding claims, **characterized in that** the extracted water-soluble compounds are selected from proteins and peptides, water-soluble vitamins, preferably B vitamins, minerals, preferably sodium, calcium, potassium, magnesium and iron, C-phycocyanin and chlorophyll.

7. The method according to any of the preceding claims, **characterized in that** the eukaryotic microalgae are selected from Chlorella, Nannocloropsis, Dunaliella and Euglena, and **in that** the cyanobacteria are selected from spirulina (Arthrospira platensis or Spirulina maxima) and AFA (Aphanizomenon flos-aquae).

8. The method according to any of the preceding claims,
**characterized in that** it comprises an additional step of solid-liquid separation, preferably using centrifugation, of the extract obtained.

9. The method according to claim 8, **characterized in that** it comprises an additional step of filtering the liquid part obtained by separation of the extract.

10. The method according to any of the preceding claims, **characterized in that** the ratio of microalgae or cyanobacteria to aqueous solution is between 1:5 and 1:50.
